# EUROPÄISCHE PATENTSCHRIFT

(11) **EP 0 611 747 B1**
(45) Veröffentlichungstag und Bekanntmachung des Hinweises auf die Patenterteilung: **14.01.1998**
(21) Anmeldenummer: 94101868.1
(22) Anmeldetag: 08.02.1994
(51) Int. Cl.: C07C 69/34, C07C 69/40, C07C 69/82, D06M 13/224, D06M 15/53, C07C 69/36, C07C 69/42, C07C 69/44, C07C 69/48, C07C 69/60, C07C 69/80

(54) **Esterverbindungen, Verfahren zu ihrer Herstellung und ihre Verwendung**
Ester compounds, their process of preparation and their use
Ester composés, leur procédé de fabrication et leur utilisation

(30) Priorität: 13.02.1993 DE 4304354
(43) Veröffentlichungstag der Anmeldung: 24.08.1994
(73) Patentinhaber: Clariant GmbH, 65929 Frankfurt am Main (DE)
(72) Erfinder: Weinelt, Frank, Dr., D-84508 Burgkirchen (DE); Jaeckel, Lothar, D-65439 Flörsheim (DE); Balekdijan, Ohannes, Dr., D-61350 Bad Homburg (DE)

(56) Entgegenhaltungen:
- GB-A- 861 963
- GB-A- 1 055 641
- US-A- 4 339 236

## Beschreibung

Die Erfindung betrifft Esterverbindungen, ein Verfahren zu ihrer Herstellung und ihre Verwendung als Faserpräparationsmittel.

GB-A-1 055 641 beschreibt Esterverbindungen aus einer Dicarbonsäure und einem Diol der Formel HOR(OR)ₘOH, worin m 1 bis 10 ist und R ein Ethylen, Propylen- oder Butylen-Diradikal ist. Bei diesen Diolen handelt es sich um solche, die nur eine Art von Oxalkylen-Einheiten im Molekül enthalten, das heißt nur Oxethylen-, nur Oxpropylen- oder nur Oxbutylen-Einheiten. Diese Diole bestehen also aus jeweils gleichen Oxalkylen-Einheiten und sind demnach reine Ethylenglykole, Propylenglykole oder Butylenglykole (vergleiche auch Beispiele I und II der GB-A-1 055 641). Diese Dicarbonsäureester werden zur Herstellung von Hydraulikflüssigkeiten verwendet. Im Unterschied zu solchen Dicarbonsäureestern enthalten die erfindungsgemäßen Ester grundsätzlich zwei Arten von Oxalkylen-Einheiten im Molekül, und zwar einen verzweigten Oxalkylenrest als "Kernmolekül", an dem beidseitig jeweils Oxethylen-Einheiten angelagert sind. Die Verwendung dieser strukturell speziellen Diole wird durch GB-A-1 055 641 keinesfalls nahegelegt.

Aus US-A-4 179 544 und US-A 4 227 390 sind Polyoxyalkylenglykole bekannt, die bei einer Wärmebehandlung von präparierten Synthesefasern, wie dem Texturieren, rückstandlos verdampfen, so daß bei deren Verwendung in Faserpräparationsmitteln die Reinigungsintervalle der eingesetzten Texturiereinrichtungen zeitlich relativ lange auseinander liegen.

Aus EP-B-162 530 sind sogenannte endverschlossene Polyoxyalkylenglykole bekannt, die gute Eigenschaften als Faserpräparationsmittel besitzen und sich ebenfalls durch geringe Rückstandsbildung auf der Faser nach Erhitzungsprozessen auszeichnen.

All diese Verbindungen weisen jedoch den Nachteil auf, daß sie biologisch nur mäßig abbaubar sind und dadurch die Abwässer ökologisch belasten. In letzter Zeit verstärkten sich die Forderungen nach biologisch leicht abbaubaren Faserpräparationsmitteln oder Textilhilfsmitteln. Diese Forderungen zielen darauf ab, die in die Abwässer von Textilbetrieben gelangenden Faserpräparationsmittel durch biologischen Abbau zu eliminieren. Unter dem Begriff "biologisch abbaubar" ist zu verstehen, daß die Faserpräparationsmittel auf biologischem Weg, zum Beispiel durch die im Klärschlamm einer Kläranlage enthaltenen Enzyme oder Bakterien, abgebaut werden. Dabei ist es wünschenswert, daß bei dem Abbau chemisch einfache Verbindungen wie Kohlendioxid, Wasser, Sulfat oder Phosphat entstehen. Die Bestimmung der biologischen Abbaubarkeit kann mit verschiedenen anerkannten Testverfahren erfolgen. Ein geeignetes Verfahren ist der sogenannte Flußwassertest (OECD-301E-Test) und der Zahn/Wellenstest (OECD-302B-Test).

In EP-A-538 714 werden ethoxylierte Diole als leicht biologisch abbaubare Faserpräparationsmittel vorgeschlagen. Diese Verbindungen können aber die Lücke nicht ganz füllen. Liegt nämlich ihr Molekulargewicht über 1200, so sind sie nicht mehr ganz leicht biologisch abbaubar. Andererseits sind die beschriebenen oxethylierten Diole mit Molmassen unter 1200 als Texturierpräparation problematisch aufgrund ungenügender Druckstabilität, wodurch sie das Filament bei der hohen mechanischen Beanspruchung während des Texturierprozesses nicht genügend schützen. Die Folge sind Produktionsablaufstörungen und Qualitätseinbußen beim Texturieren in Form von Flusen, Faden- und Kapillarbrüchen. Ein weiterer Nachteil von niedrig oxethylierten Diolen und Ethylenoxid/Propylenoxid-Verbindungen ganz allgemein ist ihre Neigung, Polyurethanmaterialien anzuquellen, die an Texturiermaschinen in Form von Rollen, Riemchen oder Texturierscheiben vorliegen, was zu frühzeitigem Verschleiß der Aggregate führt. Der Maßstab für die Polyurethanverträglichkeit einer Texturierpräparation ist in Fachkreisen die Gewichtszunahme (Quellung) des Polyurethanmaterials ®Simritan 80 AU 991 (® = eingetragenes Warenzeichen der Firma Freudenberg) nach siebentägiger Lagerung in der zu prüfenden Präparation bei 90 °C, wobei die Gewichtszunahme maximal 10 % betragen soll. Erwähnt sei ferner, daß mit steigendem Molekulargewicht der genannten oxethylierten Diole und Ethylenoxid/Propylenoxid-Verbindungen das Quellverhalten zwar zurückgeht, die biologische Abbaubarkeit aber abnimmt.

Es wurde überraschenderweise gefunden, daß spezielle Esterverbindungen gute Faserpräparationsmittel und auch leicht biologisch abbaubar sind.

Die erfindungsgemäßen Esterverbindungen sind gekennzeichnet durch die nachstehende allgemeine Formel I 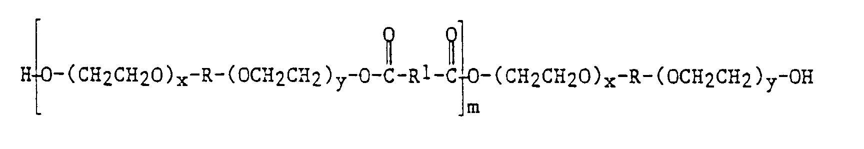 worin
- R: für einen einfach oder mehrfach, vorzugsweise ein- oder zweifach, alkylsubstituierten Alkylenrest mit 2 bis 4 Kohlenstoffatomen in der Alkylenkette und mit Methyl, Ethyl, Propyl oder Isopropyl als Alkylsubstituent steht, wobei Methyl bevorzugt ist,
- x: plus y eine Zahl von 2 bis 35 ist, wobei weder x noch y Null ist,
- R¹: für -(CH₂)_{z}- steht, worin z Null oder eine ganze Zahl von 1 bis 12 ist, oder für einen Phenylenrest oder Vinylenrest steht, und
- m: eine Zahl von 1 bis 30 ist.

Bevorzugte Verbindungen der Formel I sind solche, wobei
- R: der 1-Methylethylenrest (Isopropylenrest), 1-Methylpropylenrest, 2-Methylpropylenrest cder der 2,2-Dimethylpropylenrest ist, wobei der Isopropylenrest besonders bevorzugt ist,
- x: plus y, das heißt die Summe von x und y, eine Zahl von 5 bis 22 ist, wobei weder x noch y Null ist,
- R¹: -(CH₂)_{z}- ist, worin z 1 bis 8 ist, oder ein Phenylenrest oder Vinylenrest ist, und
- m: eine Zahl vcn 1 bis 10 ist.

Die Oligoester der Formel I sind erhältlich durch Veresterung eines Diols der nachstehenden Formel II

HO-(CH₂CH₂O)ₓ-R-(OCH₂CH₂)_{y}-OH

worin R, x und y die in Formel I angegebene Bedeutung haben,
mit einer Dicarbonsäure der nachstehenden Formel III

HOOC-R¹-COOH

worin R¹ die in Formel I angegebene Bedeutung hat, im Molverhältnis von 1 zu 0,25 bis 1, vorzugsweise 1 zu 0,45 bis 1.

Als Diole oder Polyethylenglykole kommen vorzugsweise solche der Formel II in Betracht, die eine Molmasse von 200 bis 1500 aufweisen, vorzugsweise von 300 bis 1000. Besonders bevorzugte Diole sind jene, wobei R Isopropylen ist, das sind jene mit Isopropylenglykol als Startverbindung. Solche Polyethylenglykole und ihre Herstellung sind in EP 0 166 958 B1 beschrieben. Sie werden bevorzugt in der Weise hergestellt, daß die dem Rest R in Formel II entsprechende Glykolverbindung, zum Beispiel Ethylenglykol oder Isopropylenglykol, vorgelegt und mit derjenigen Molmenge Ethylenoxid umgesetzt wird, die der angestrebten Summe x plus y in Formel II entspricht. Im einzelnen wird in der Regel so vorgegangen, daß die Glykolverbindung und ein alkalischer Katalysator in einem mit Rührer ausgestatteten Druckgefäß vorgelegt werden, worauf zur Erzeugung einer inerten Atmosphäre vorzugsweise mit Stickstoff gespült wird. Gegebenenfalls anwesendes Wasser wird unter Anwendung von Vakuum ausgetragen. Nun wird bei einer Temperatur von 110 bis 170 °C, vorzugsweise 120 bis 160 °C, flüssiges oder gasförmiges Ethylenoxid bei dem sich einstellenden Druck zudosiert und angelagert. Das Ende der Ethylenoxid-Addition ist am abgefallenen und im wesentlichen konstant bleibenden Druck erkennbar. Zur Entfernung von gegebenenfalls anwesenden flüchtigen Anteilen wird das erhaltene Polyethylenglykol unter Vakuum 0,3 bis 1 Stunde lang auf etwa 80 bis 120 °C gehalten. Bevorzugte alkalische Katalysatoren sind Natriumhydroxid, Natriumcarbonat oder Natriummethylat sowie die entsprechenden Kaliumverbindungen. Die Menge an Katalysator beträgt im allgemeinen 0,1 bis 5 Gew.-%, vorzugsweise 0,3 bis 3 Gew.-%, bezogen auf die Menge an eingesetzter Glykolverbindung.

Die einzusetzenden Dicarbonsäuren können aromatischer oder aliphatischer Art sein. Die aliphatischen Dicarbonsäuren können gesättigt oder ungesättigt sein. Die aromatische Dicarbonsäure ist vorzugsweise die Phthalsäure (Benzol-ortho-dicarbonsäure), die Terephthalsäure (Benzol-para-dicarbonsäure) und die Isophthalsäure (Benzol-meta-dicarbonsäure). Die aliphatische gesättigte Dicarbonsäure ist vorzugsweise eine mit C₁ bis C₈-Alkylengruppen, wie Malonsäure, Bernsteinsäure, Glutarsäure, Adipinsäure, Pimelinsäure, Suberinsäure, Azelainsäure und Sebacinsäure. Die aliphatische ungesättigte Dicarbonsäure ist vorzugsweise die Fumarsäure oder die Maleinsäure. Von den genannten Dicarbonsäuren sind die aliphatischen gesättigten Dicarbonsäuren sowie die Maleinsäure bevorzugt. Es versteht sich von selbst, daß anstelle der Dicarbonsäuren auch Derivate davon eingesetzt werden können, zum Beispiel die entsprechenden Anhydride und Halogenide, wobei die Anhydride (Dicarbonsäureanhydride) bevorzugt sind.

Die Veresterung der Diole der Formel II mit den Dicarbonsäuren oder Dicarbonsäureanhydriden gemäß Formel III wird vorzugsweise ohne Lösungsmittel, das heißt in Substanz, und unter Abdeckung mit einem Schutzgas durchgeführt, wobei das Reaktionswasser aus der Reaktionsmischung abdestilliert wird. Die Veresterungstemperaturen liegen bei 170 bis 230 °C, vorzugsweise bei 180 bis 210 °C. Die Veresterungsreaktion wird vorzugsweise in saurer oder schwachsaurer Katalyse durchgeführt. Saure Katalyse erhält man zum Beispiel mit p-Toluolsulfonsäure, Methansulfonsäure oder unterphosphoriger Säure oder Mischung davon als Katalysator. Schwachsaure Katalyse erhält man zum Beispiel, wenn man als Katalysator eine molare Mischung im Verhältnis von 1 zu 1 von unterphosphoriger Säure und NaOH (fest oder in Form einer zum Beispiel 1 bis 50gew.%igen wäßrigen Lösung) einsetzt. Ein besonders bevorzugter Katalysator ist eine Mischung aus p-Toluolsulfonsäure oder Methansulfonsäure und unterphosphoriger Säure im Gewichtsverhältnis von 1 zu 0,5, wenn die unterphosphorige Säure als solche (das heißt 100%ig) eingesetzt wird, oder im Gewichtsverhältnis von 1 zu 1, wenn die unterphosphorige Säure in Form einer etwa 50gew.%igen wäßrigen Lösung eingesetzt wird. Mit diesem Katalysator werden Oligoester mit besonders guter Farbqualität und ohne Niederschlag erhalten. Die Menge an Katalysator kann in weiten Grenzen variieren. Sie beträgt im allgemeinen 0,03 bis 0,5 Gew.-%, bezogen auf das Gewicht von Diol und Dicarbonsäure oder Dicarbonsäureanhydrid, vorzugsweise 0,05 bis 0,2 Gew.-%.

Das Ende der Veresterungsreaktion ergibt sich aus der Säurezahl des Veresterungsproduktes, die kleiner 5, vorzugsweise kleiner 3 und besonders bevorzugt 0,3 bis 2,5, sein soll. Das erhaltene Veresterungsprodukt, das nach Abkühlung auf Raumtemperatur gegebenenfalls einer Filtration unterworfen wird, stellt das angestrebte Faserpräparationsmittel dar. Das Produkt hat eine Viskosität im allgemeinen von größer 500 mPa · s bei 20 °C, vorzugsweise von 700 bis 5000 und besonders bevorzugt von 900 bis 3000 mPa · s bei 20 °C. Weitere Kennzahlen für die erfindungsgemäßen Oligoester sind die OH-Zahl (Hydroxylgruppenzahl), die Verseifungszahl und die Iodfarbzahl. Die OH-Zahl liegt im allgemeinen bei 10 bis 160, vorzugsweise 70 bis 115, die Verseifungszahl bei 40 bis 300, vorzugsweise 50 bis 220, und die Iodfarbzahl bei < 0,1 bis 20, vorzugsweise < 0,1 bis 8 und besonders bevorzugt bei < 0,1 bis 1.

Die erfindungsgemäßen Esterprodukte können als solche, das heißt allein, oder in Mischung mit anderen an sich bekannten Faserpräparationsmitteln bei der Herstellung und/oder Behandlung von Fasern eingesetzt werden. Das erfindungsgemäße Faserpräparationsmittel besteht also aus bis zu 100 Gew.-% an Esterverbindungen der Formel I oder anders ausgedrückt, das erfindungsgemäße Mittel ist gekennzeichnet durch einen Gehalt oder besteht aus Esterverbindungen der Formel I. Als Mischungskomponenten können die bekannten und üblichen Mittel eingesetzt werden wie Ethylenoxid/Propylenoxid-Polymere, Tenside, Esteröle aus Fettalkoholen und Fettsäuren, Phosphorsäureester und deren Salze, Oxethylate von niedrigen Alkanolen oder Alkandiolen, Fettalkoholoxethylate, Fettaminoxethylate und/oder Fettsäureoxethylate, die zum Beispiel als Gleitmittel, Netzmittel, Emulgatoren, Fadenschlußmittel und/oder Antistatika dienen. In den Mischungen mit anderen Mitteln liegt die Menge an Verbindungen der Formel I im allgemeinen bei 5 bis 95 Gew.-%, vorzugsweise bei 30 bis 60 Gew.-%, Gewichtsprozente bezogen auf das (fertige) Faserpräparationsmittel. So besteht eine bevorzugte erfindungsgemäße Mischung im wesentlichen aus
a) 30 bis 60 Gew.-% von einem Esterprodukt der Formel I,
b) 5 bis 20 Gew.-% von einem gesättigten oder ungesättigten, linearen oder verzweigten C₈ bis C₁₈-Alkohol ethoxyliert mit 5 bis 15 Ethylenoxid-Einheiten und
c) 20 bis 50 Gew.-% von einem Diol der oben angegebenen Formel II, Gewichtsprozente bezogen auf die (fertige) Mischung.

Beim Präparieren von Fasern mit den erfindungsgemäßen Esterprodukten oder mit Mischungen davon oder mit Mischungen der erfindungsgemäßen Esterprodukte mit anderen bekannten Faserpräparationsmitteln wird auf der Faser eine Menge von im allgemeinen 0,1 bis 1 Gew.-% aufgebracht, vorzugsweise eine Menge von 0,3 bis 0,5 Gew.-%, Gewichtsprozente bezogen auf das Gewicht der (behandelten) Faser. Das Fasermaterial besteht vorzugsweise aus Polyestern, Polyamiden, Polyacrylnitrilen, Polyolefinen oder aus Copolymeren davon. Das Aufbringen der erfindungsgemäßen Mittel auf das Fasermaterial wird bevorzugt in der Weise durchgeführt, daß man die Faser mit dem Mittel besprüht oder daß man die Faser durch ein Bad führt, in dem das Mittel als solches oder in Form einer vorzugsweise 10 bis 30gew.%igen Lösung, Emulsion oder Dispersion vorliegt.

Das erfindungsgemäße Oligoesterprodukt weist eine Reihe von Vorteilen auf. Es ist unerwartet gut biologisch abbaubar. Dies ist um so mehr überraschend, als es sich um strukturell komplexe und relativ hochmolekulare Verbindungen handelt. Es bildet ferner nur wenig Rückstände bei Erhitzungsprozessen und bringt Polyurethane nur sehr wenig zum Quellen. Bei Verwendung als Faserpräparationsmittel weisen die beschriebenen Esterprodukte also die geforderten Eigenschaften auf; sie bewirken auch die besonders erwünschte fadenschützende Elastizität. Die erfindungsgemäßen Esterprodukte und ihre Mischungen besitzen also gleichzeitig die Vorzüge einer leichten biologischen Abbaubarkeit bei geringer Quellung von Polyurethanmaterialien verbunden mit geringer Rückstandneigung und der erforderlichen fadenschützenden Druckelastizität. Daher eignen sie sich gut als Faserpräparationsmittel, vorzugsweise als Texturierpräparation und als Texturierpräparationskomponente. Die erfindungsgemäßen Oligoester sind im allgemeinen wasserlöslich oder in Wasser dispergierbar.

Die Erfindung wird nun an Beispielen noch näher erläutert.

### Herstellung der erfindungsgemäßen Esterverbindungen:

### Beispiel 1

In einem Reaktionsgefäß mit Thermometer, Wasserabscheider mit Rückflußkühler, Rührer und Heizeinrichtung wurden 600 g (1 mol) von einem Diol der Formel II mit R = Isopropylen und x plus y = 12 (Molmasse 600), 50 g (0,5 mol) Bernsteinsäureanhydrid und 0,9 g (0,14 Gew.-%, bezogen auf die Gewichtssumme aus Diol und Bernsteinsäureanhydrid) von einem Gemisch aus p-Toluolsulfonsäure und unterphosphoriger Säure im Gewichtsverhältnis von 1 : 0,5 als Katalysator vorgelegt. Die vorgelegte Mischung wurde unter Stickstoffatmosphäre auf 200 °C erhitzt und bei einer Temperatur von 190 bis 210 °C unter kontinuierlicher Wasserabscheidung gehalten (etwa 6 Stunden), bis das Veresterungsprodukt eine Säurezahl von etwa 0,7 hatte, und damit nach Abkühlung das erfindungsgemäße Oligoesterprodukt vorlag.

### Beispiele 2 bis 16

Unter analogen Reaktionsbedingungen wie in Beispiel 1 wurden weitere erfindungsgemäße Produkte hergestellt.

In der nachstehenden Tabelle I sind die Beispiele 1 bis 16 zusammengefaßt, wobei die Ausgangsverbindungen, Diol und Dicarbonsäure, das eingesetzte Molverhältnis von Diol zu Dicarbonsäure und die folgenden Eigenschaften des erhaltenen Oligoesterproduktes angegeben sind: Die Säurezahl (SZ), die Hydroxylgruppenzahl (OHZ), die Verseifungszahl (VZ), die Iodfarbzahl (IFZ) und die Viskosität (VSK) in mPa · s bei 20 °C. Die eingesetzten Diole sind in Tabelle I mit zum Beispiel "PEG600PR" (Beispiel 1) oder "PEG300PR" (Beispiel 3) bezeichnet, was ein Polyethylenglykol (PEG) mit der Molmasse 600 beziehungsweise 300 bedeutet, wobei die Startverbindung Isopropylenglykol (PR) ist. Die in Tabelle I mit zum Beispiel "PEG300ET" (Beispiel 5) oder "PEG600ET" (Beispiel 6) bezeichneten Diole sind Polyethylenglykole (PEG) mit der Molmasse 300 beziehungsweise 600, wobei die Startverbindung Ethylenglykol (ET) ist. Von den eingesetzten Diolen ist auch noch die Summe x plus y gemäß Formel II angegeben.

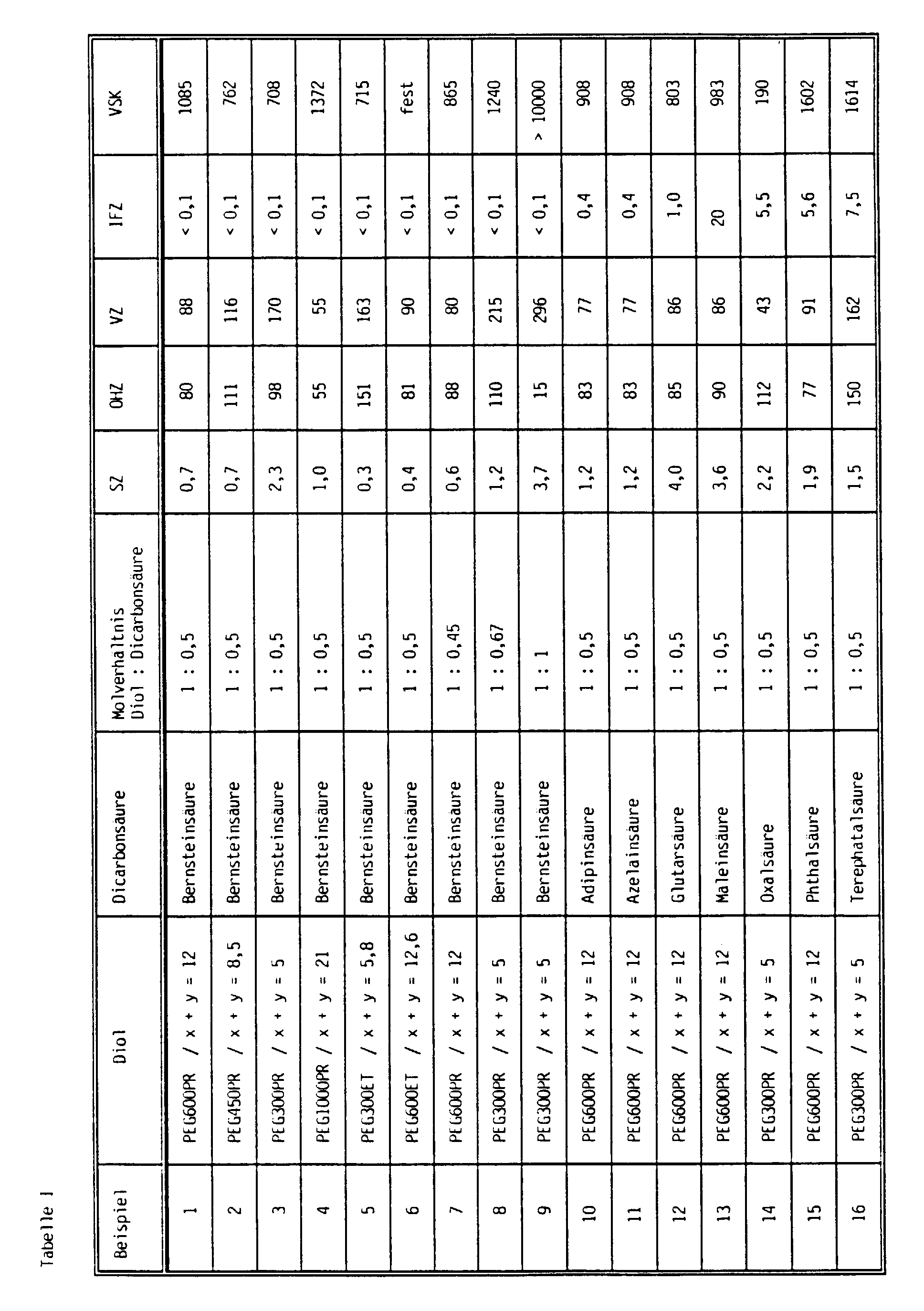

### Prüfung der erfindungsgemäßen Esterverbindungen im Hinblick auf Faserpräparationsmittel:

Von den in den Beispielen 1 bis 16 hergestellten Esterprodukten wurden - stellvertretend für alle anderen - jene der Beispiele 1, 3 und 5 bezüglich Quellung von Polyurethan, Verdampfung und biologische Abbaubarkeit getestet; das Esterprodukt des Beispiels 1 wurde auch bezüglich Texturierung getestet.
- Prüfung der Quellung von Polyurethanmaterial
®Simritan 80 AU 991 (® = eingetragenes Warenzeichen der Firma Freudenberg):
Es wurde die Gewichtszunahme ermittelt, die nach Lagerung von Proben des genannten Polyurethans 7 Tage lang bei 90 °C im Esterprodukt der Beispiele 1, 3 und 5 eintrat. Ein Vergleichstest wurde mit dem in Beispiel 1 eingesetzten Diol durchgeführt. Die Ergebnisse sind nachstehend zusammengefaßt:

| Beispiel | Gewichtszunahme |
|---|---|
| 1 | 7 % |
| 3 | 6 % |
| 5 | 6 % |
| Vergleich | 15 % |

- Prüfung der Verdampfungsrate:
Im Abdampftest wurde jeweils 1 g vom Esterprodukt der Beispiele 1, 3 und 5 bei 220 °C gehalten und der Verlust nach 0,33 Stunden (20 Minuten) und 24 Stunden, ausgedrückt in Gewichtsprozent, bestimmt. Die Ergebnisse sind nachstehend zusammengefaßt:

| Beispiel | 20 Minuten | 24 Stunden |
|---|---|---|
| 1 | 5 % | > 90 % |
| 3 | 4 % | > 90 % |
| 5 | 3 % | > 90 % |

- Prüfung auf biologische Abbaubarkeit:
Die biologische Abbaubarkeit wurde mit Hilfe des OECD-301E-Testes und des OECD-302B-Testes ermittelt (Bestimmung der biologischen Elimination in Abhängigkeit von der Zeit). Nachstehend sind die Höchstwerte der biologischen Abbaubarkeit nach 28 Tagen für die Esterprodukte der Beispiele 1, 3 und 5 angegeben:

| Beispiel | OECD-301E-Test | OECD-302B-Test |
|---|---|---|
| 1 | 80 % | 100 % |
| 3 | 64 % | 100 % |
| 5 | 71 % | 100 % |

- Prüfung der Eignung als Texturierpräparation:
Es wurde ein Texturiertest mit drei erfindungsgemäßen Produkten durchgeführt, und zwar mit dem Esterprodukt von Beispiel 1 (Produkt 1), mit einer Mischung aus 85 Gew.-% Esterprodukt von Beispiel 1 und 15 Gew.-% von einem Fettalkohol ethoxyliert mit 8 mol Ethylenoxid (Produkt 2) und mit einer Mischung aus 30 Gew.-% Esterprodukt von Beispiel 5, 20 Gew.-% von einem Fettalkohol ethoxyliert mit 8 mol Ethylenoxid und 50 Gew.-% von dem in Beispiel 1 eingesetzten oxethylierten Diol (Produkt 3), und zum Vergleich dazu wurde ein übliches Polyoxyalkylenglykol und das in Beispiel 1 eingesetzte oxethylierte Diol getestet.

Die Testprodukte wurden jeweils beim Ausspinnen einer Polyesterfaser mit Hilfe einer Zahnradpumpe auf die Faser appliziert. Im folgenden sind die Testdurchführung und die Texturierdaten im einzelnen angegeben:
- Texturiermaschine: Barmag FK 6/700
- Spinntiter: Polyester, POY (preorientated yarn) 260dtex f32 matt
- Auftrag: 0,35 Gew.-%
- Geschwindigkeit: 620 m pro Minute
- Spindel: Friktionsaggregat, Keramikscheiben
- DR (Streckverhältnis): 1,59
- D/y (Umfangsgeschwindigkeit der
   Friktionsscheiben dividiert durch
   die Zuliefergeschwindigkeit des Fadens): 2,2
- Temperatur der beiden Heizeinrichtungen: 205 und 180 °C
- T₁/T₂ (Fadenspannung vor dem Aggregat und Fadenspannung nach dem Aggregat): 62 cN und 65 cN.

Die Tests wurden danach beurteilt, wieviel Störungen insgesamt in Form von Flusen, Fadenbrüchen und Kappilarbrüchen bei 100 km Faserherstellung auftraten. Nachstehend ist die Anzahl der Störungen bei den getesteten Produkten angegeben:

| Testprodukt | Anzahl der Störungen |
|---|---|
| Produkt 1 | 10 |
| Produkt 2 | 8 |
| Produkt 3 | 4 |
| Polyoxyethylenglykol | 21 |
| oxethyliertes Diol | 260 |

Wie die Testergebnisse zeigen, besitzen die erfindungsgemäßen Esterprodukte eine hervorragende Eigenschaftskombination im Hinblick auf Faserpräparation.

## Patentansprüche

1. Esterverbindungen der nachstehenden allgemeinen Formel I 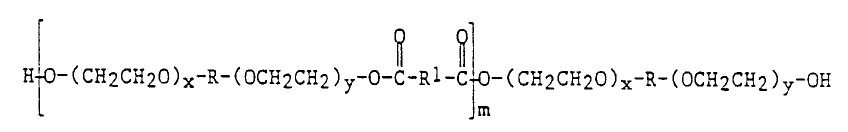 worin
R für einen einfach oder mehrfach alkylsubstituierten Alkylenrest mit 2 bis 4 Kohlenstoffatomen in der Alkylenkette und mit Methyl, Ethyl, Propyl oder Isopropyl als Alkylsubstituent steht,
x plus y eine Zahl von 2 bis 35 ist, wobei weder x noch y Null ist,
R¹ für -(CH₂)_{z}- steht, worin z Null oder eine ganze Zahl von 1 bis 12 ist, oder für einen Phenylenrest oder Vinylenrest steht, und
m eine Zahl von 1 bis 30 ist.

2. Esterverbindungen nach Anspruch 1, wobei R für einen ein- oder zweifach alkylsubstituierten Alkylenrest mit 2 bis 4 Kohlenstoffatomen in der Alkylenkette und mit Methyl als Alkylsubstituent steht.

3. Esterverbindungen nach Anspruch 1, wobei R der 1-Methylethylenrest, 1-Methylpropylenrest, 2-Methylpropylenrest oder der 2,2-Dimethylpropylenrest ist, x plus y eine Zahl von 5 bis 22 ist, wobei weder x noch y Null ist, R¹ -(CH₂)_{z}- ist, worin z 1 bis 8 ist, oder ein Phenylenrest oder Vinylenrest ist, und m eine Zahl von 1 bis 10 ist.

4. Esterverbindungen nach einem der Ansprüche 1 bis 3, wobei R der 1-Methylethylenrest ist.

5. Verfahren zur Herstellung der Esterverbindungen gemäß Anspruch 1, gekennzeichnet durch Veresterung eines Diols der nachstehenden Formel II
HO-(CH₂CH₂O)ₓ-R-(OCH₂CH₂)_{y}-OH
worin R, x und y die in Formel I angegebene Bedeutung haben,
mit einer Dicarbonsäure der nachstehenden Formel III
HOOC-R¹-COOH
worin R¹ die in Formel I angegebene Bedeutung hat, im Molverhältnis von 1 zu 0,25 bis 1.

6. Verfahren nach Anspruch 5, wobei das Diol und die Dicarbonsäure im Molverhältnis von 1 zu 0,45 bis 1 eingesetzt werden.

7. Verfahren nach Anspruch 5 oder 6, wobei die Veresterung bei einer Temperatur von 170 bis 230 °C und bis zu einer Säurezahl des Veresterungsproduktes von kleiner 5 durchgeführt wird.

8. Verfahren nach Anspruch 5 oder 6, wobei die Veresterung bei einer Temperatur von 170 bis 230 °C und bis zu einer Säurezahl des Veresterungsproduktes von kleiner 3 durchgeführt wird.

9. Faserpräparationsmittel enthaltend oder bestehend aus mindestens einer Esterverbindung gemäß Anspruch 1.

10. Mittel nach Anspruch 9, enthaltend 5 bis 95 Gew.-% von mindestens einer Esterverbindung gemäß Anspruch 1, Gewichtsprozente bezogen auf das Mittel.

11. Mittel nach Anspruch 9, enthaltend 30 bis 60 Gew.-% von mindestens einer Esterverbindung gemäß Anspruch 1, Gewichtsprozente bezogen auf das Mittel.

12. Mittel nach Anspruch 9, bestehend im wesentlichen aus
a) 30 bis 60 Gew.-% von mindestens einer Esterverbindung gemäß Anspruch 1,
b) 5 bis 20 Gew.-% von einem Ethoxylat mit 5 bis 15 Ethylenoxid-Einheiten von einem C₈ bis C₁₈-Alkohol und
c) 20 bis 50 Gew.-% von einem Diol der im Anspruch 5 angegebenen Formel II, Gewichtsprozente bezogen auf das Mittel.

## Claims

1. An ester compound of the formula I below 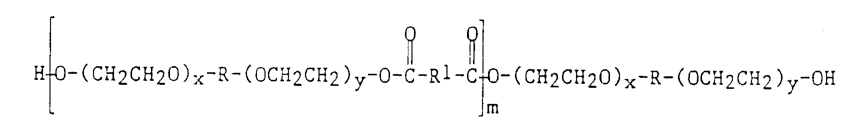 in which
R is an alkylene radical which has 2 to 4 carbon atoms in the alkylene chain and is substituted by one or more alkyl substituents, the alkyl substituent being methyl, ethyl, propyl or isopropyl,
x plus y is 2 to 35, neither x nor y being zero,
R¹ is -(CH₂)_{z}-, in which z is zero or an integer from 1 to 12, or is a phenylene radical or vinylene radical, and
m is 1 to 30.

2. An ester compound as claimed in claim 1, R being an alkylene radical which has 2 to 4 carbon atoms in the alkylene chain and is substituted by 1 or 2 alkyl substituents, the alkyl substituent being methyl.

3. An ester compound as claimed in claim 1, R being an 1-methylethylene radical, 1-methylpropylene radical, 2-methylpropylene radical or 2,2-dimethylpropylene radical, x plus y being 5 to 22, neither x nor y being zero, R¹ being -(CH₂)_{z}-, in which z is 1 to 8, or being a phenylene radical or vinylene radical, and m being 1 to 10.

4. An ester compound as claimed in one of claims 1 to 3, R being a 1-methylethylene radical.

5. A process for preparing an ester compound as claimed in claim 1, which comprises esterifying a diol of the formula II below
HO-(CH₂CH₂O)ₓ-R-(OCH₂CH₂)_{y}-OH
in which R, x and y have the meaning given in formula I
with a dicarboxylic acid of the formula III below
HOOC-R¹-COOH
in which R¹ has the meaning given in formula I in a molar ratio of 1:0.25 to 1.

6. The process as claimed in claim 5, wherein the diol and the dicarboxylic acid are used in a molar ratio of 1:0.45 to 1.

7. The process as claimed in claim 5 or 6, wherein esterification is carried out at a temperature of 170 to 230°C and up to an acid number of the esterification product of less than 5.

8. The process as claimed in claim 5 or 6, wherein esterification is carried out at a temperature of 170 to 230°C and up to an acid number of the esterification product of less than 3.

9. A spin finish containing or being composed of at least one ester compound as claimed in claim 1.

10. A spin finish as claimed in claim 9, containing 5 to 95% by weight of at least one ester compound as claimed in claim 1, % by weight being based on the spin finish.

11. A spin finish as claimed in claim 9, containing 30 to 60 % by weight of at least one ester compound as claimed in claim 1, % by weight being based on the spin finish.

12. A spin finish as claimed in claim 1, being composed essentially of
a) 30 to 60% by weight of at least one ester compound as claimed in claim 1,
b) 5 to 20% by weight of an ethoxylate obtained by ethyoxylating a C₈-C₁₈-alcohol with 5 to 15 ethylene oxide units, and
c) 20 to 50% by weight of a diol of the formula II given in claim 5, % by weight being based on the spin finish.

## Revendications

1. Composé ester de formule générale I ci-après 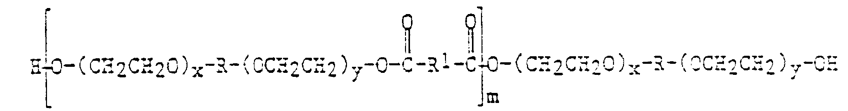 dans laquelle
R est un reste alkylène comportant 2 à 4 atomes de carbone dans la chaîne alkylène, substitué une ou plusieurs fois, de préférence une ou deux fois, par alkyle, avec méthyle, éthyle, propyle ou isopropyle en tant que substituant alkyle, en donnant la préférence au méthyle,
x plus y est un nombre de 2 à 35, ni x ni y n'étant pas zéro,
R¹ représente -(CH₂)_{z}-, dans laquelle z est zéro ou un nombre entier de 1 à 12, ou un reste phénylène, ou un reste vinylène, et
m est un nombre de 1 à 30.

2. Composés ester selon la revendication 1, R étant un reste alkylène avec 2 à 4 atomes de carbone dans la chaîne alkylène substitué une ou deux fois par alkyle, et méthyle étant substituant alkyle.

3. Composés ester selon la revendication 1, où R représente le reste 1-méthyléthylène, le reste 1-méthylpropylène, le reste 2-méthylpropylène ou le reste 2,2-diméthylpropylène, x plus y, c'est-à-dire la somme de x et y, est un nombre de 5 à 22, ni x ni y n'étant pas zéro, R¹ représente -(CH₂)_{z}-, dans laquelle z va de 1 à 8, ou un reste phénylène, ou un reste vinylène, et m est un nombre de 1 à 10.

4. Composés ester selon l'une des revendications 1 à 3, où R est le reste 1-méthyléthylène.

5. Procédé pour la préparation des composés esters selon la revendication 1, caractérisé par estérification d'un diol de formule II ci-après
HO-(CH₂CH₂O)ₓ-R(OCH₂CH₂)_{y}-OH
dans laquelle R, x et y ont la signification donnée dans la formule I,
avec un acide dicarboxylique de formule III suivante :
HOOC-R¹-COOH
dans laquelle R¹ a la signification donnée dans la formule I,
dans un rapport molaire de 1:0,25 à 1.

6. Procédé selon la revendication 5, où le diol et l'acide dicarboxylique sont utilisés dans un rapport molaire de 1:0,45 à 1.

7. Procédé selon la revendication 5 ou 6, où l'estérification est mise en oeuvre à une température de 170 à 230 °C et on la conduit jusqu'à un indice d'acide du produit d'estérification inférieur à 5.

8. Procédé selon la revendication 5 ou 6, où l'estérification est mise en oeuvre à une température de 170 à 230 °C et jusqu'à un indice d'acide du produit d'estérification inférieur à 3.

9. Agent de préparation de fibres contenant ou constitué d'au moins un composé ester selon la revendication 1.

10. Agent selon la revendication 9, contenant 5 à 95 % en poids d'au moins un composé ester selon la revendication 1, les pourcentages en poids sont relatifs à l'agent.

11. Agent selon la revendication 9 contenant 30 à 60 % en poids d'au moins un composé ester selon la revendication 1, les pourcentages en poids sont relatifs à l'agent.

12. Agent selon la revendication 9, constitué essentiellement de
a) 30 à 60 % en poids d'au moins un composé ester selon la revendication 1,
b) 5 à 20 % en poids d'un éthoxylate avec 5 à 15 motifs oxyde d'éthylène d'un alcool en C₈ à C₁₈ et
c) 20 à 50 % en poids d'un diol de formule II indiqué dans la revendication 5, les pourcentages en poids sont relatifs à l'agent.
